# EUROPEAN PATENT APPLICATION

(11) **EP 1 043 318 A1**
(43) Date of publication of application: **11.10.2000**
(21) Application number: 97954963.1
(22) Date of filing: 19.11.1997
(51) Int. Cl.: C07D 239/62, C07D 239/64, C07D 239/66, C07D 239/68, A61K 31/515

(54) **SALTS OF 5,5'-ARYLIDENEBISBARBITURIC AND 5,5'-ARYLIDENEBIS(2-THIOBARBITURIC) ACIDS AND 5,5'-ARYLIDENEBIS(2-THIOBARBITURIC) ACIDS HAVING AN ANTIBACTERIAL, ANTI-CHLAMYDIAL, ANTIVIRAL AND IMMUNO-MODULATING ACTIVITY**

(71) Applicant: Tets, Viktor Veniaminovich, St. Petersburg, 191025 (RU)
(72) Inventor: ASHKINAZI, Rimma Iliinichna, St.Petersburg, 191025 (RU)
(74) Representative: Mercer, Christopher Paul
(86) International application number: RU9700372
(87) International publication number: WO9925699

(57) **Abstract**

The present invention relates to the production of new chemical substances having an anti-microbial, antiviral, immuno-modulating and anti-tumoral activity. This invention more precisely relates to the synthesis of new substances consisting of 5,5'-arylidenebis(2-thiobarbituric) acids and in salts of 5,5'-arylidenebisbarbituric and 5,5'-arylidenebis(2-thiobarbituric) acids of general formula (I) where X is oxygen or sulphur. R¹ is hydrogen, a nitro group or an alkoxy group, R² is hydrogen, a nitro group, an alkoxy group or halogen and Cat⁺ is a proton or a pyridin- or 2-hydroxyethylammonium-cation. In the preferred embodiments of these substances, the following conditions are observed: 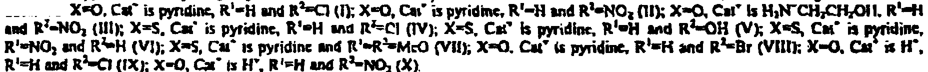 This invention also relates to eight synthesis instances of these substances, to three tables representing the chemical and physical characteristics of the substances thus obtained and to the results of five series of experiments. These synthesis instances, tables and results demonstrate the biological properties of these substances on myco-bacteria, on herpes viruses and on Chlamydia trachomatis as well as their interferon-inducing activity and the absence of acute toxicity.

## Description

### Field of the invention

The invention relates to the area of organic chemistry and medicine, specifically to barbituric acids and their derivatives, which are designed for use as substances having antiviral activity against the herpes virus, and as substances having antichlamydic and immunomodulating activity.

Many derivatives of pyridine are biologically active substances. The spectrum of biological activity of compounds of this class is extremely broad. The derivatives of pyrimidine are nucleic bases (uracil, thymine, cytosine), vitamins (thiamine, phosphothiamine), coenzymes (cocarboxylase), and they are utilized as pharmaceuticals having soporific, anticonvulsive (barbiturates, hexamidine, benzonal), diuretic (mercusal), antiinflammatory (pentoxil, methyluracil), and antithyroidal (methylthiouracil) activities; they are synthetic analogs of vitamins ("bephothiamine"), anabolic (orotic acid), anti-inflammatory and antibacterial (sulfazin, sulfadimethazin, sulfamonomethoxin, sulfadimethoxin, bactrim, salazodimethoxin), antimalarial (chloridin), anticarcinogenic (dopan, phosphamide, ethimidide, fluorouracil, fluorofur, cytarabin) substances [1, 2].

In recent decades, systems in which the pyrimidine ring is condensed with other heterocycles have received considerable attention. Such heterocycles are frequently analogs of natural, biologically active substances. They include purines, which occur in natural and synthetic biologically active substances, nucleic acids (adenine, guanine), ATP, substances that excite the central nervous system (caffeine, theobromine, theophylline, "nuhexine," diprophylline, xanthinol nictotunate), substances used in suppressing tissue incompatibility in organ transplants (azathioprine) (cytostatic and immunodepressive effect), and substances having an anabolic (inosine) or antileukemic effect (mercaptopurine), pyrazolo[3,4-d]pyramidines used to treat diseases accompanied by hyperuremia (allopurinol); pteridines, used as diuretics (triamterene), vitamins (riboflavin, folic acid), anticarcinogenic drugs (methotrexate), pyrmido[5,4-d]pyrimidines having vasodilational properties (dipyridamole [1, 2].

Data on the biological activity of the most varied derivatives of 5-ylidenebarbituric acids have been summarized in a review [3] which notes the anticoma, antimicrobial, spasmolytic, antipyretic and antitumor activity of these substances. The material of this review can be supplemented by more recent data on the pesticidal, insecticidal [4], antipyrene [5, 6], analgesic [7], antiviral [8-10] and fungicidal [11-13] activities exhibited by compounds of this group of chemical substances.

Thus, literature data on the biological activity of 5-substituted barbituric acids and condensed systems containing the pyrimidine ion fragment, as stated above, indicate that they have pesticidal, insecticidal, fungicidal activity, antipyretic, anti-tumor, antiviral, antimicrobial, anti-allergenic activity, and act on the cardiovascular and immune systems. The effectiveness of most of the substances that have been studied, however, is not satisfactorily high, and many of them are toxic and cause side-effects. Furthermore, most microbes responsible for human and animal diseases are resistant to the most important of the existing drugs. This resistance makes it impossible for them to be used against such dangerous infections as tuberculosis, herpes, and chlamydiosis [14-16]. The high resistance of tumor cells to existing anti-tumor drugs is also well known [17].

The literature contains only a few examples of the condensation of barbituric and 2-thiobarbituric acid with carbonyl compounds at a ratio of 2:1 [18-22]. For example, barbituric acid reacts with isatin and 1-methylisatin to form bisbarbiturylisatin [18], 2-thiobarbituric acid when heated with benzaldehyde in aniline converts to the dianyline salt of 5,5'-benzylidenebis(2-thiobarbituric) acid [19], and when 5,5'-salicylidenebis(2-thiobarbituric) acid is heated in ethanol it converts, according to [19-22], into 5,5'-salicylidenebis(2-thiobarbituric) acid. It was shown in [23, 24], however, that barbituric and 2-thiobarbituric acids give 5-(4,6-dioxo-5-pyrimidinyl)chromeno[2,3-d]pyrimdines with salicylic aldehydes. The description of 5,5'-ylidenebisbarbituric or 5,5'-ylidenebis(2-thiobarbituric) acids being synthesized from aliphatic aldehydes and ketones [6, 25] as well as that of 5,5'-benzylidenebisbarbituric acid [26] are, most probably, in error.

5,5'-Salicylidenebis(2-thiobarbituric) acid is also known. Its chemical structure and properties have been described in detail in [27]. It is of low toxicity and prolongs sleep induced by barbiturates, but not other symptoms, and also has a vasopressive effect. No other biological activity has been found for it. This last compound is closest to those claimed here in terms of its chemical nature. It has therefore been selected as the prototype of the invention.

The material above suggests the potential usefulness of conducting a scientific search and of developing effective new biologically active substances by condensing barbituric acids with carbonyl compounds, producing, specifically, 5,5'-arylidenebis(2-thiobarbituric) acids or salts of 5,5'-arylidenebisbarbituric acids and of 5,5'-arylidenebis(2-thiobarbituric) acids.

### Object of the invention

The object of the invention is to create a new group of chemical substances having antimicrobial, antiviral, immunomodulating and antitumor activities.

### Essence of the invention

The object of the invention is achieved by the synthesis of novel compounds - 5,5'-arylidenebis(2-thiobarbituric) acids or salts of 5,5'-arylidenebisbarbituric acids and of 5,5'-arylidenebis(2-thiobarbituric) acids of the general formula 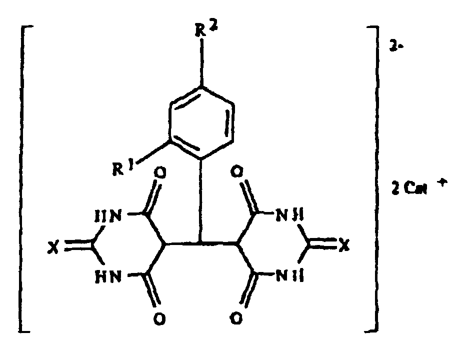 where
X is selected from the group OXYGEN, SULFUR,
R¹ is selected from the group HYDROGEN, the NITRO GROUP, or an ALKOXY GROUP,
R² is selected from the group HYDROGEN, the NITRO GROUP, the HYDROXYL GROUP, an ALKOXY GROUP, HALOGEN,
Cat⁺ is selected from the group PROTON, or a PYRIDINE or 2-HYDROXYETHYLAMMONIUM CATION.

The best variants of the claimed compounds known to the inventor as of the date of delivery of the application are when:

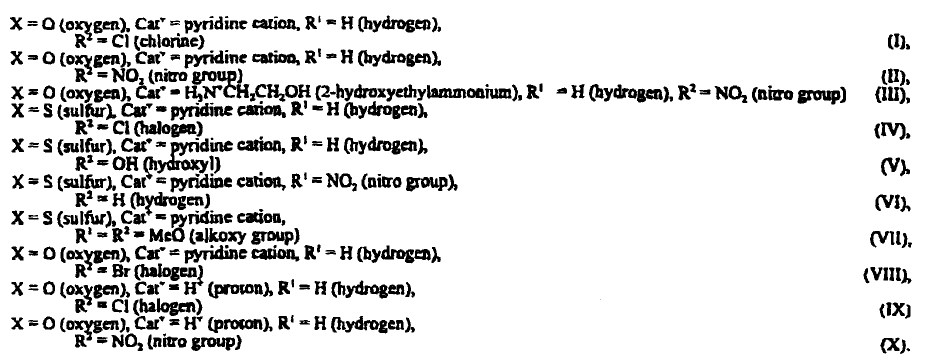

It should be noted that the use of other representatives of the alkoxy group or halogens does nor differ in principle in the synthesis process or in the biological activities of the substances obtained, i.e., it is not the specific elements that are of importance, but that they belong to the groups cited in the general formula. The proposed best variants [preferred embodiments] are listed in the table that follows for clarity.

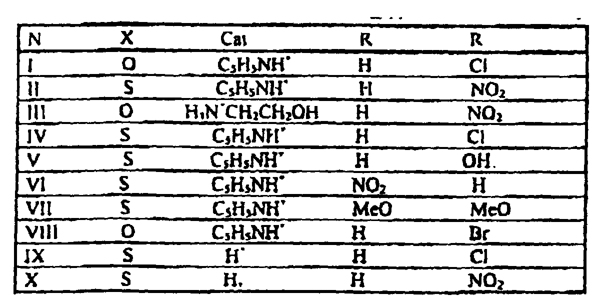

The general formula specified above and all of the specific embodiment enumerated are novel, and they are unknown to the applicants from available sources of information.

### Disclosure of the invention

The essence of the invention is explained below by eight general examples of the synthesis of the claimed substances, three tables of the physicochemical characteristics of the substances obtained and the results of five series of experiments to determine their comparative biological properties (with corresponding tables of quantitative measurements), where:
**Example 1 - variant of the synthesis of pyridine salts of 5,5'-arylidenebisbarbituric and 5,5'-arylidenebis(2-thiobarbituric) acids (I, II, IV-VIII).**
**Example 2** **- variant of the synthesis of pyridine salts of 5,5'-arylidenebisbarbituric and 5,5'-arylidenebis(2-thiobarbituric) acids (I, II, IV-VIII).**
**Example 3** **- variant of the synthesis of the 2-hydroxyethylammonium salt of 5,5'-(*p*-nitrobenzylidene)barbituric acid (III).**
**Example 4** **- variant of the synthesis of 5,5'-arylidenebis(2-thiobarbituric) acids (IX, X).**
**Example 5** **- best example known to the inventors of the synthesis for the pyridine salt of 5,5'-(*p*-bromobenzylidene)barbituric acid (VIII).**
**Example 6** **- best example known to the inventors of the synthesis for the pyridine salt of 5,5'-(*p*-chlorobenzylidene)bis(2-thiobarbituric acid) (IV).**
**Example 7** **- best example known to the inventors of the synthesis of the 2-hydroxyethylammonium salt of 5,5'-(*p*-nitrobenzylidene)bisbarbituric acid (III).**
**Example 8** **- best example known to the inventors of the synthesis of 5,5'-(*****p*****-nitrobenzylidene)bis(2-thiobarbituric) acid (X).**
**Table 1** - PMR Spectra of solutions of the substances obtained in DMSO-d₆ (δ, ppm).
**Table 2** - NMR ¹³C Spectra of solutions of salts of 5,5'-arylidene bisbarbituric acids and of 5,5'-arylidene(2-thiobarbituric) acids in DMSO-d₆ (δ, ppm).
**Table 3** - Yields, temperatures of decomposition and elemental analyses of 5,5'-arylidenebis(2-thiobarbituric acids) and of salts of 5,5'-arylidenebisbarbituric acids and 5,5'-arylidenebis(2-thiobarbituric) acid
**Experiment 1 - Determination of the activity of compounds on pathogenic microbacteria.**
**Experiment 2** **- Determination of the acute toxicity of the claimed compounds.**
**Experiment 3** **- Determination of the activity of the claimed compounds on the herpes virus.**
**Experiment 4** **- Determination of the interferon-inducing activity of the claimed compounds.**
**Experiment 5** **- Determination of the activity of the claimed compounds on Chlamydia trachomatis.**

### Example 1 - variant of the synthesis of pyridine salts of 5,5'-arylidenebisbarbituric and 5,5'-arylidenebis(2-thiobarbituric) and 5,5'-arylidenebis(2-thiobarbituric) acids (I, II, IV-VIII).

A mixture of 4.6 mmol of 5-arylidenebarbituric acid and 4.6 mmol of barbituric acid in 10-15 ml of pyridine is refluxed for 1-2 hours. After the reaction mixture cools, the precipitate is filtered, washed with acetone and dried at 55-60°C at a residual pressure of 30 mm Hg. To isolate the thio-derivatives from the reaction mixture, 10-20 ml of dioxane or water are added to the mixture, the precipitate that forms is filtered, washed with ethanol and dried.

### Example 2 - variant of the synthesis of pyridine salts of 5,5'-arylidenebisbarbituric and 5,5'-arylidenebis(2-thiobarbituric) and 5,5'-arylidenebis(2-thiobarbituric) acids (I, II, IV-VIII).

A mixture of 3 mmol of barbituric acid and 15 mmol of the corresponding aldehyde in 10-15 ml of pyridine is refluxed for 1-2 hours. After the reaction mixture cools, the precipitate is filtered, washed with acetone and dried at 55-60°C at a residual pressure of 30 mm Hg. To isolate the thio-derivatives from the reaction mixture, 10-20 ml of dioxane or water are added to the mixture, the precipitate that forms is filtered, washed with ethanol and dried. To carry out the reaction, 3 mmol of barbituric acid and 1.5 mmol of the corresponding aldehyde are used. The synthesis and isolation of the product are performed in a manner analogous to the preceding technique

### Example 3 - variant of the synthesis of the 2-hydroxyethylammonium salt of 5,5'-(p-nitrobenzylidene)bisbarbituric acid (III).

2 g of the pyridine salt of 5,5'-(*p*-nitrobenzylidene)bisbarbituric acid are added to a solution of 0.4 g of 2-aminoethanol in 50 ml of ethanol. The mixture is stirred at 20°C for 10 min. The bright red precipitate that forms is filtered, washed with ethanol 3x40 mL, dried at 50°C under vacuum. The yield is quantitative.

### Example 4 - variant of the synthesis of 5,5'-arylidenebis(2-thiobarbituric) acids (IX, X).

To a mixture of 1 g of 2-thiobarbituric acid and 10 ml of ethanol there is added a solution of 0.5 g of benzaldehyde in 10 ml of ethanol. After the reaction mixture is stirred at 20°C, for 20-30 min, the precipitate that forms is filtered and dried. The precipitate is purified by heating in acetone, in which 5,5'-arylidenebis(2-thiobarbituric) acids are not soluble.

### Examples of the best examples known to the inventors [preferred embodiments] of the claimed compounds of formula (I).

### Example 5 - best example known to the inventors of the synthesis for the pyridine salt of 5,5'-(p-bromobenzylidene)bisbarbituric acid (VIII).

A mixture of 4.6 mmol of 5-*p*-bromobenzylidenebarbituric acid and 4.6 mmol of barbituric acid in 10-15 ml of pyridine is refluxed for 1-2 hours. After the reaction mixture cools, the precipitate is filtered, washed with acetone and dried at 55-60°C at a residual pressure of 30 mm Hg

### Example 6 - best example known to the inventors of the synthesis of the pyridine salt of 5,5'-(p-chlorobenzylidene)bis(2-thiobarbituric) acid (IV).

A mixture of 3 mmol of 2-thiobarbituric acid and 1.5 mmol of *p*-chlorobenzaldehyde in 10-15 ml of pyridine is refluxed for 1-2 hours. After the reaction mixture cools, 10-20 ml of dioxane or water is added, the precipitate that forms is filtered, washed with ethanol and dried at 55-60°C

### Example 7 - best example known to the inventors of the synthesis of the 2-hydroxyethylammonium salt of 5,5'-(p-nitrobenzylidene)bis barbituric acid.

2 g of the pyridine salt of 5,5'-(*p*-nitrobenzylidene)bis barbituric acid are added to a solution of 0.4 g of 2-aminoethanol in 50 ml of ethanol. The mixture is stirred at 20°C for 10 min. The bright red precipitate that forms is filtered, washed with ethanol 3x40 mL, dried at 50°C under vacuum. The yield is quantitative.

### Example 8 - best example known to the inventors of the synthesis of 5,5'-(p-nitrobenzylidene)bis (2-thiobarbituric) acid (X).

To a mixture of 1 g of 2-thiobarbituric acid and 10 ml of ethanol there is added a solution of 0.5 g of benzaldehyde in 10 ml of ethanol. After the reaction mixture is stirred at 20°C, for 20-30 min, the precipitate that forms is filtered and dried. The precipitate is purified by heating in acetone, in which 5,5'-arylidenebis(2-thiobarbituric) acids are not soluble.

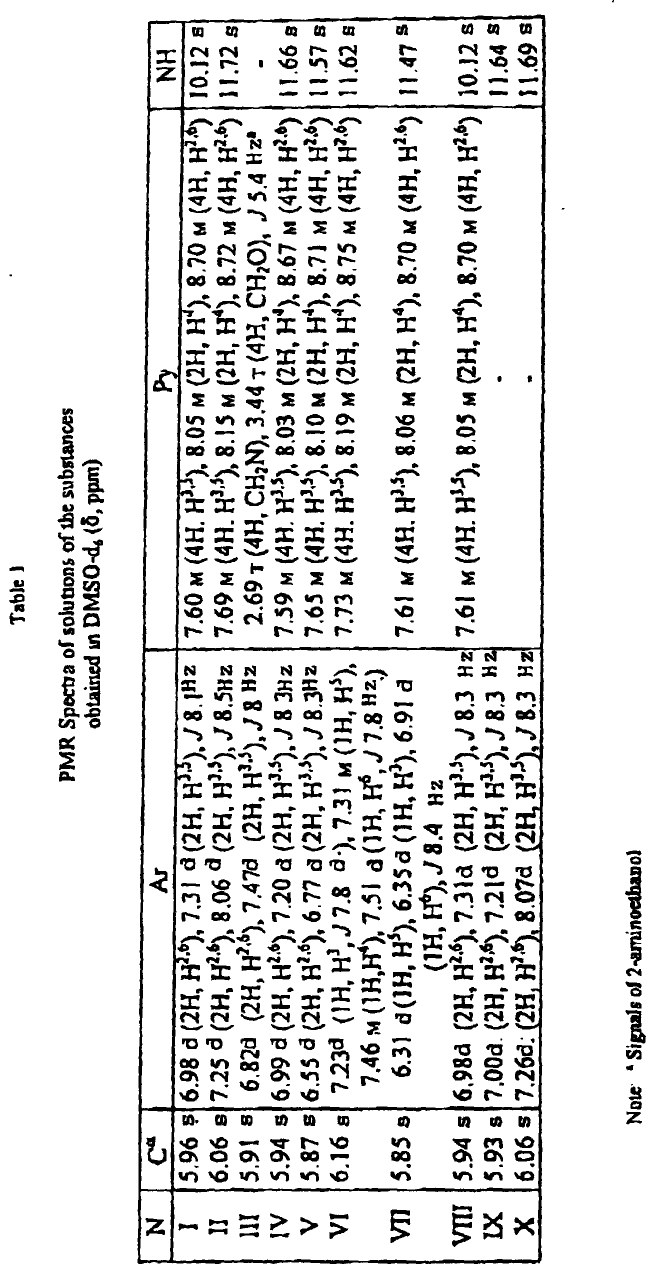

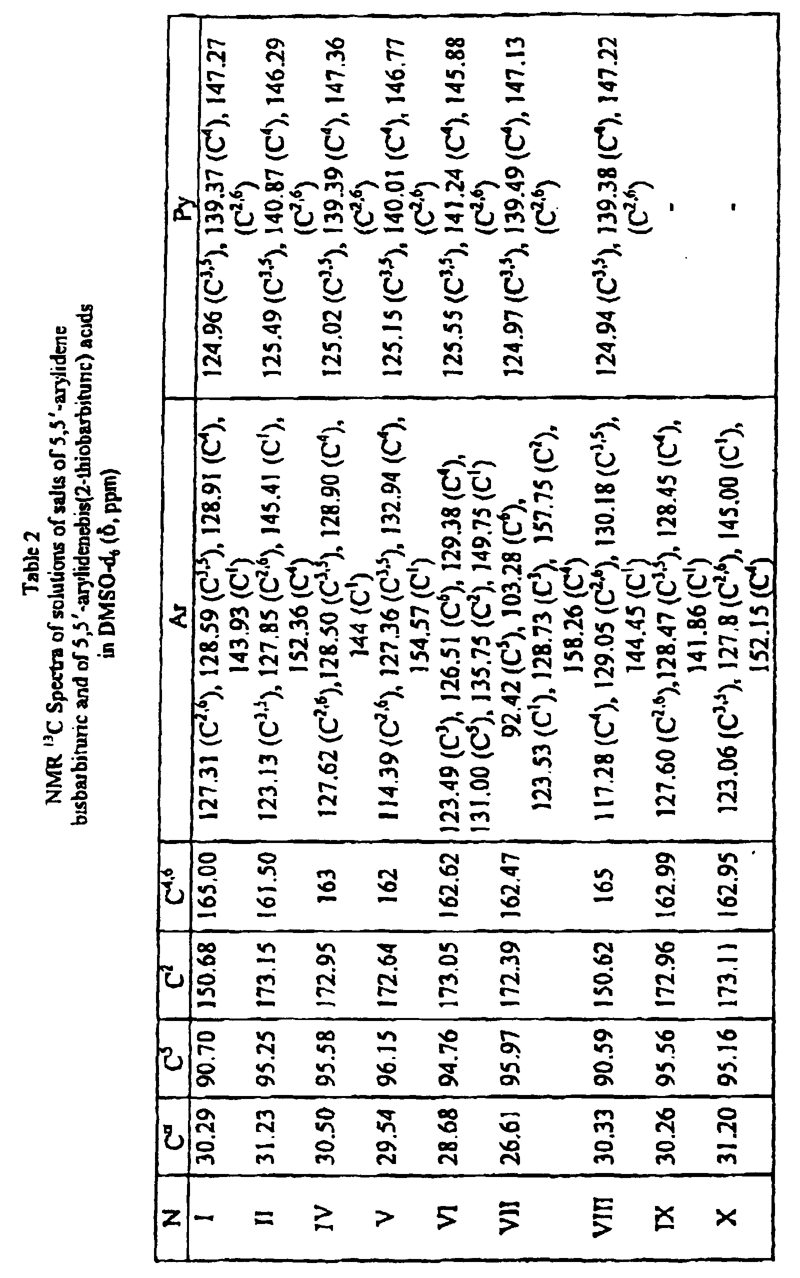

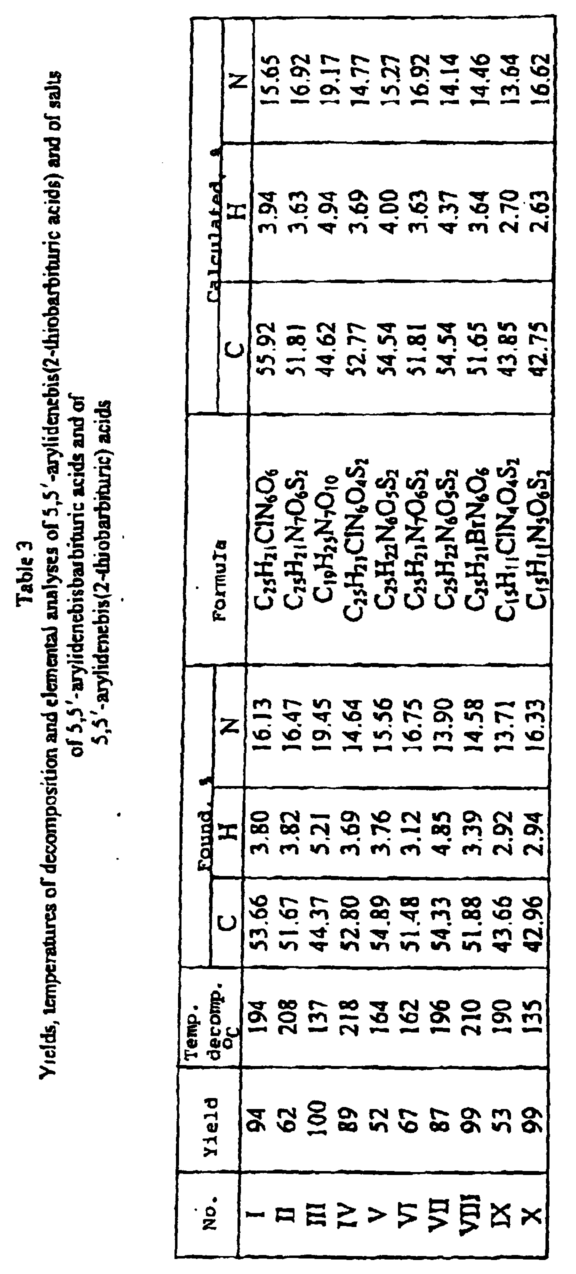

### Experimental tests of the biological activity of the claimed compounds

### Experiment 1 - Determination of the activity of the compounds on pathogenic bacteria

In the experiment we used strains isolated from patients at clinics in St. Petersburg (Russia) in 1996; M. tuberculosis 61 (S), which is sensitive to the action of known antimicrobial drugs (aminoglycosides, rifampicin, isoniazid, ethambutol; M. tuberculosis 16R, which is resistant to the basic antimicrobial drugs. In the experiments we used Lewenstein-Jensen medium. The preparations were dissolved in DMSO and added to individual rest tubes with the medium in a final concentration of 10 mg/l. After the test strain was inoculated on the surface of the medium, the test tubes were incubated for 30 days. In the control we used DMSO in corresponding concentrations and standard preparations used in treating tuberculosis and microbacterioses
(Table 4)

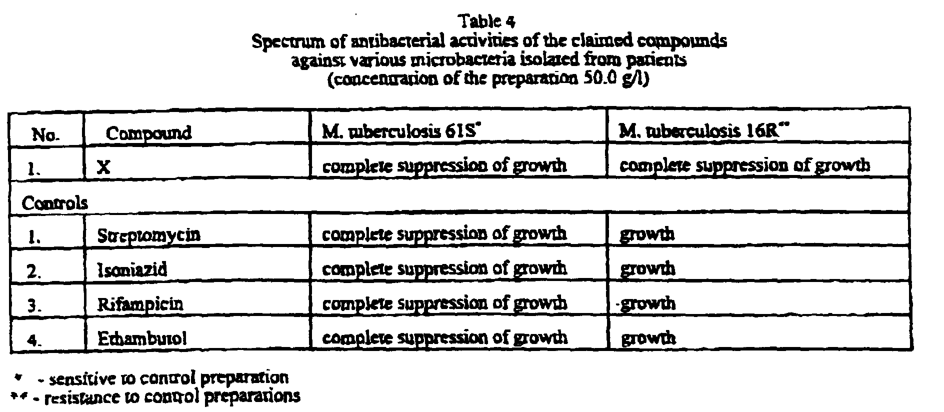

The data indicate that the claimed compounds enumerated in the table suppress the growth of all species and strains of microbacteria used in the experiment. Thus, *in vitro* they also act on strains of microbacteria isolated from patients that are resistant to the basic preparations used in treating tuberculosis. The remaining claimed compounds exhibit less antimicrobial activity under analogous conditions.

### Experiment 2. Determination of acute toxicity of the claimed compounds

Acute toxicity was determined on non-linebred white mice weighing 18-20 g. The claimed compounds were prepared in a range of concentrations: 1500, 700, 500, 100, 20 and 5 µg/kg. Five animals were used to study each specific concentration of a compound. The preparation was administered once a day by mouth of intravenously. The observation period was 14 days On days 1, 8 and 15 the animals in every group were weighed. For a control we used animals who received the emulsion without the test compounds. All animals, both those that died in the course of the experiment and those that survived to the end of the experiment, were opened to allow macroscopic examination of internal organs. In the course of the experiment no loss of weight was observed or any change in behavior or external appearance, nor was there any loss of life. Based on the results of the macroscopic examination of internal organs of the animals in the test and the control groups, no pathological findings were made, [text missing] The acute toxicity (LD50) of the claimed compound - compound X - 1000 mg/kg The data show that when the claimed compounds were taken by mouth or intravenously at a dosage level of 1000 mg/kg, no acute toxicity was found for mice.

### Experiment 3. Determination of the activity of claimed compounds on the herpes virus

The antiviral activity was studied relative to the virus herpes simplex type 1 (VPG-1/Leningrad/248/88) by a standard method [21]. The virus was grown on a reinoculated Vero cell culture obtained from the cell culture bank of the Institute of Cytology of the Russian Academy of Sciences.

### Procedure

To cells grown on medium RPMI-1640 with 10% calf serum and placed in the wells of a 96-well plate, the virus was added to give a final concentration of 10 particles/ml and the claimed compounds dissolved in DMSO were added to give a final concentrations of 100, 10, and 1 mg/l. We used 5 individual wells for every concentration tested. The plate was incubated for 60 min at 38°C in a CO₂ incubator. After incubation the virus was removed and new medium was added that contained the claimed compounds in the test concentrations. The results were evaluated according to the existence of a cytopathic action of the virus on the cells after 36 hours of incubation at 38°C in the CO₂ incubator.

The following controls were used in the experiment:
1. Control of culture cells (capability for normal growth).
2. Control of virus (assessment of reproductive capability).
3. Control of antiviral activity of an antiviral preparation - acylcovir.
4. Control of compounds (toxicity of compounds).
5. Control of solvent (DMSO) on toxicity.

To access the cytopathic effect of the virus, we counted the number of unchanged cells in 100 fields formed by a specialized grid on the eyepiece of the inversion microscope. The results are presented in Table 5.

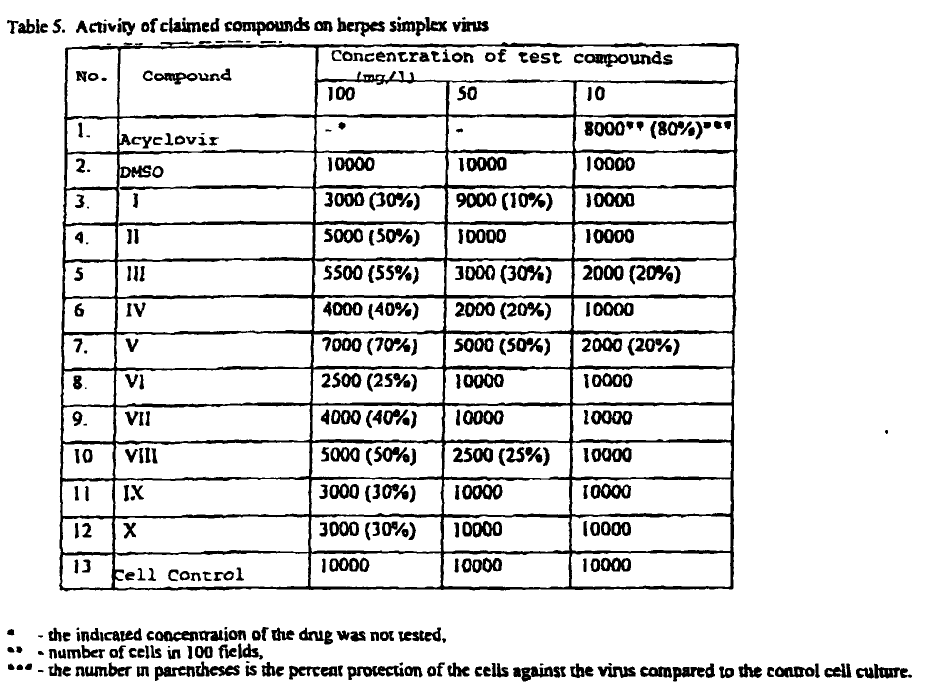

The results indicate that all of the claimed compounds exhibit antiherpes activity comparable to that of the standard drug acyclovir.

### Experiment 4. Determination of interferon-inducing activity of claimed compounds.

Interferon synthesis induction by the claimed preparations was conducted on a primary culture of humans lymphocytes (the cells in the human body that are the main producers of interferon). To obtain a culture of the lymphocytes we used fresh blood (12 hours after collection) from healthy animals (not from the second group). To isolate the lymphocytes, heparinized blood obtained from a healthy donor was centrifuged in a density gradient (phycoll-verographin) of 1.71 g/cm³ to isolate the fraction of the immunocompetent cells. This fraction was taken and diluted with RPM1-1640 nutrient medium containing 5% bovine fetal serum, 0.3 mg/ml of L-glutamine, 100 units/ml of penicillin, and 50 mg/ml of strepromycin. The lymphocyte concentration was determined after staining with methyl blue and the number of cells in the Goryaev chamber was calculated. The starting solutions of the claimed substances were diluted with RPMI-1640 nutrient medium to give final concentrations of the substances of 100 mg/l, 10 mg/l, and 1 mg/l after the lymphocyte suspension was added. The final concentration of the lymphocytes in the induction mixture was 3·10⁶ cells/ml.
The following controls were processed in parallel with the test samples:
1) control for the spontaneous production of interferon (IFN) by lymphocytes;
2) control for the process occurring under the action of standardized IFN inducer N-methyl-N-(a,D-glucopyranosyl)ammonium 10-methylene carboxylate of acridone (cycloferon).
3) control for the process occurring under the action of standardized INF inducer Neovir (sodium 10-methylenecarboxylate-9-acridone) with the corresponding content of DMSO in the experimental samples.
4) control for the spontaneous production of interferon in the presence of DMSO in a quantity corresponding to the test samples.

The control and the experimental samples were incubated for 24 hours at 37°C. Following incubation the samples were centrifuged at 2000 g to settle out the cellular elements and the INF-containing supernatant was taken from the samples and analyzed for INF content. The cell residue was resuspended in the same volume of nutrient medium, stained with a vital dye - trypan blue - and the number of cells in the Goryaev chamber was calculated (as described above) to determine the cytotoxic activity of the preparations.
For the quantitative determination of INF in the control and experimental samples we used the ProCon IF2 Plus immunoenzyme test system sold by TOO Proteinovyi Kontur. To determine the quantity of interferon in the sample we used a solid-phase immunoenzyme method utilizing horseradish peroxidase as the enzyme indicator. The activity of the bound peroxidase was measured using an automated photometer at a wavelength of 450 nm for the microplanchets with a microprocessor. To calculate the results we determined in parallel the activity of INF in standard solutions of INF containing known amounts of the preparation. On the basis of the results obtained we plotted a calibration curve that allowed us to obtain data expressed in International Units (IU) from the microprocessor of the automated photometer. The results of the analysis are expressed in IU of activity of INF per ml in the given induction system containing 3·10° lymphocytes/ml. Every experimental and control point was studied in 4 parallel runs.
Controls of the immunoenzyme reaction.
1. Control of DMSO with the nutrient medium.
2. Control of the system components (according to Instructions).
The results obtained were analyzed statistically using the t-test and the confidence interval at p = 0.05 was calculated. Analysis of the convergence of the results of the parallel tests were performed.
As a result of the experiment it was established that all of the claimed compounds have an ability to induce INF synthesis, which demonstrates their antiviral and antitumor effectiveness (Table 6).

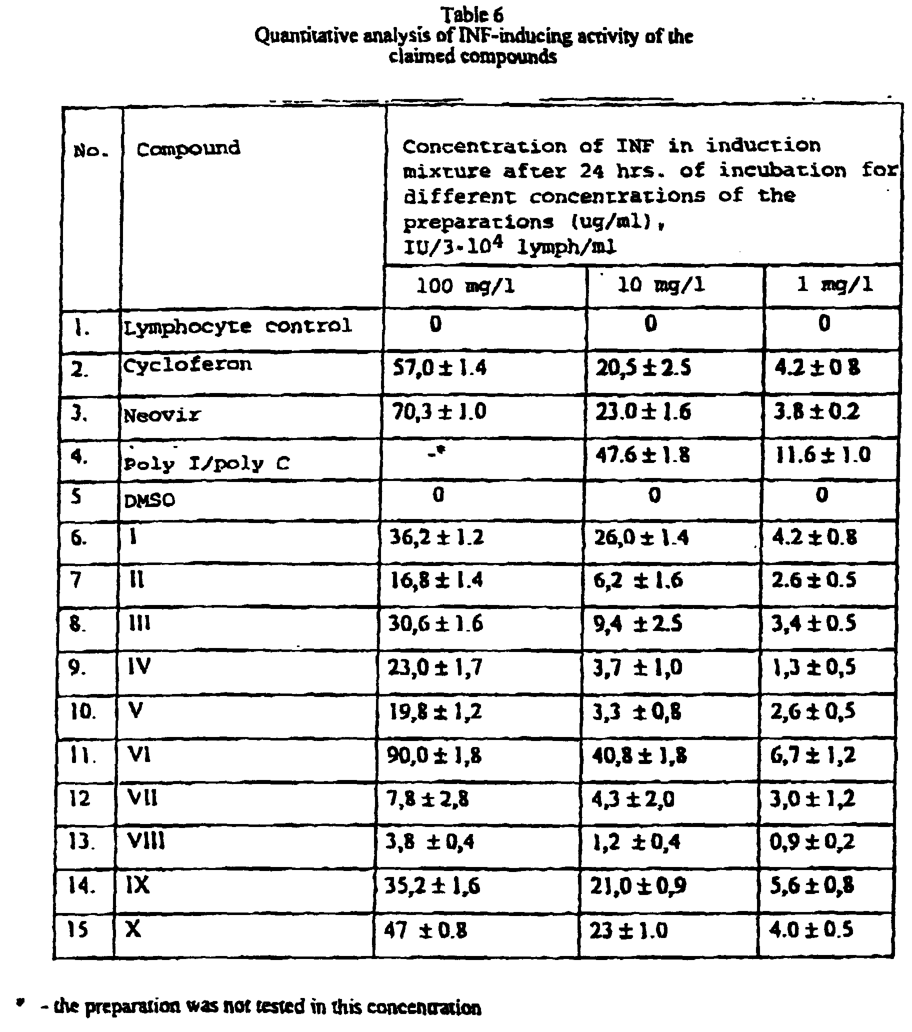

### Experiment 5. Determination of the action of the claimed compounds on Chlamydia trachomatis.

The antimicrobial activity of the claimed compounds was studied on C. trachomatis D323 - a standard strain from the collection of the Department of Microbiology of the Pavlov St.-Petersburg State University. This strain was isolated from a patient with chlamydic urethritis. It has the morphology and physiological activity characteristic of the species, and is sensitive to the action of drugs used in treating chlamydic infections. In the work reported here we used McCoy and L929 cell cultures obtained from the Institute of Cytology of the Russian Academy of Sciences.

### Procedure

The cells were grown in flasks of neutral glass in RPMI-1640 medium with an addition of 10% fetal calf serum. The experiment was placed in glass (toxicity-free) flat-bottomed flasks and covered with glass. The cells were introduced into the medium in a final concentration of 1-10 cells/ml. After a monolayer was obtain, standard infectious doses of chlamydia that had been maintained in a frozen state at -70°C were added to test tubes. At the same time, the test compounds were added to the cells to give a final concentration of 100 mg/ml. The sample was centrifuged as 2400 g for 60 min at room temperature and incubated at 37°C for 2 hours. After this, the nutrient medium was replaced by a new medium that contained 5% fetal calf serum and cycloheximide (2 µg/ml) and the claimed compounds were re-added in the same concentration. A parallel series of samples was run using medium without the cycloheximide in order to avoid its influence on the test substances. The samples were incubated for 48 hours in a CO₂ incubator.
The controls included: controls of the cell cultures, control for the action of solvents, control for the action of chlamydia in the absence of any preparations at all, control for the sensitivity of chlamydia to a standard antimicrobial preparation - cyprofloxacin [29], control of the test compounds for toxicity relative to cell cultures.

The results were evaluated from the detection of chlamydia cytoplasmatic inclusion by an immunoenzyme method (MicroTrac Chlamydia trachomatis Direct Specimen Test) and from chlamydia antigens using CylaMonoScreen (Russian-British Joint Venture, 66 Regent's Park Road, London NW1 7SX) [30, 31]. The effect of the prepartions was determined by analyzing the state of the monolayer and the number of cells with CPE based on a comparison to the control (cell culture infected with C. trachomatis D323) by counting the number of unchanged cells in 100 viewing fields produced by a specialized grid for the microscope eyepiece.
The results of the control samples satisfying the requirements of the experiment:
control of cell culture - the morphology of the cells and the state of the monolayer are appropriate to the given type of cells, control of growth of chlamydia in the cell culture - the existence of CPE in the monolayer, control of the action of a standard antimicrobial preparation - a reduction in the number of cells with CPE in the monolayer compared to the previous control, control of toxicity of claimed compounds - no toxicity, control for the activity of solvents - no toxic effect on the cells. The results of the tests are presented in Table 7.

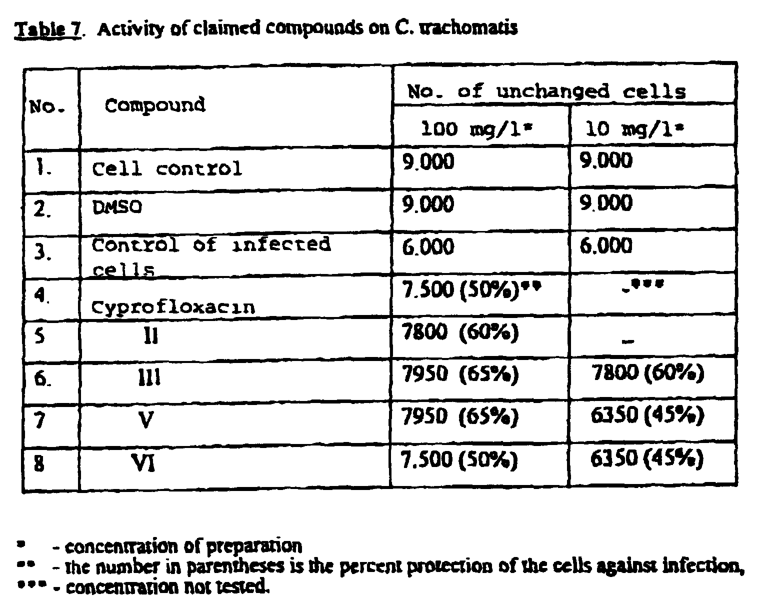

The data obtained demonstrate that the claimed compounds listed in Table 7 exhibit marked activity against chlamydia, surpassing that of cyprofloxacin, the standard drug. The remaining claimed compounds exhibit less marked activity in protecting cells against chlamydia under the conditions of the experiment.

### Industrial application

Examples 1-8 of the practical identification [sic] and analysis of the claimed compounds listed in Tables 1-3 confirm the possibility of the laboratory and industrial synthesis of all ten claimed compounds, which are capable of being manufactured by the modern pharmaceutical industry, as well as their precise identification using standard control methods
The results of the 5 series of experiments on identifying the biological properties presented showed that the claimed compounds exhibit biological activity relative to a variety of microorganisms, including microbacteria, chlamydia, herpes simplex virus, and also have interferon-inducing activity. This last suggests the possibility of using them to treat herpes, other viral and certain carcinogenic illnesses.
The facts presented above prove that the goal of the invention was achieved, namely, the synthesis of a novel class of heterocyclic compounds having a high and broad biological activity, in particular, antimicrobacterial, immunostimulating, antichlamydic and antiviral activities.
Thus, we believe that the claimed compounds (substances) satisfy all requirements for a patent; they are novel, non-obvious, and can be produced on an industrial basis.

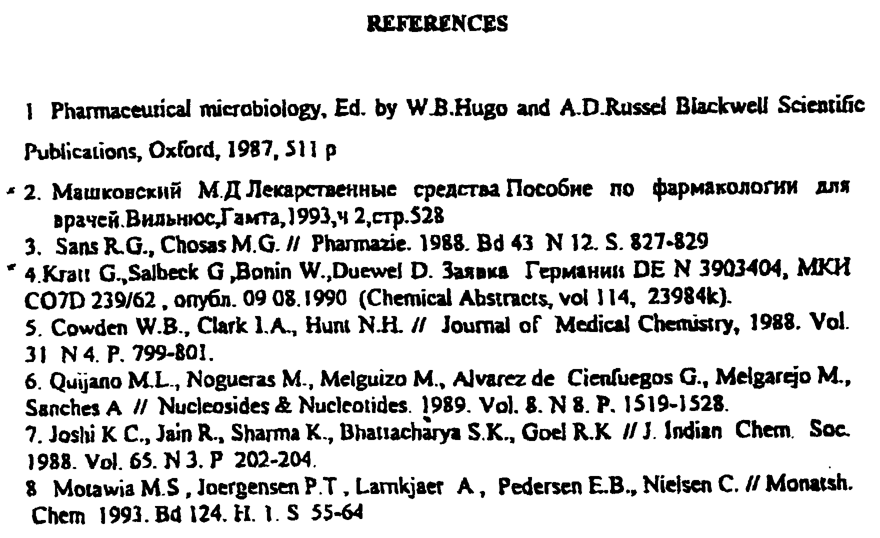

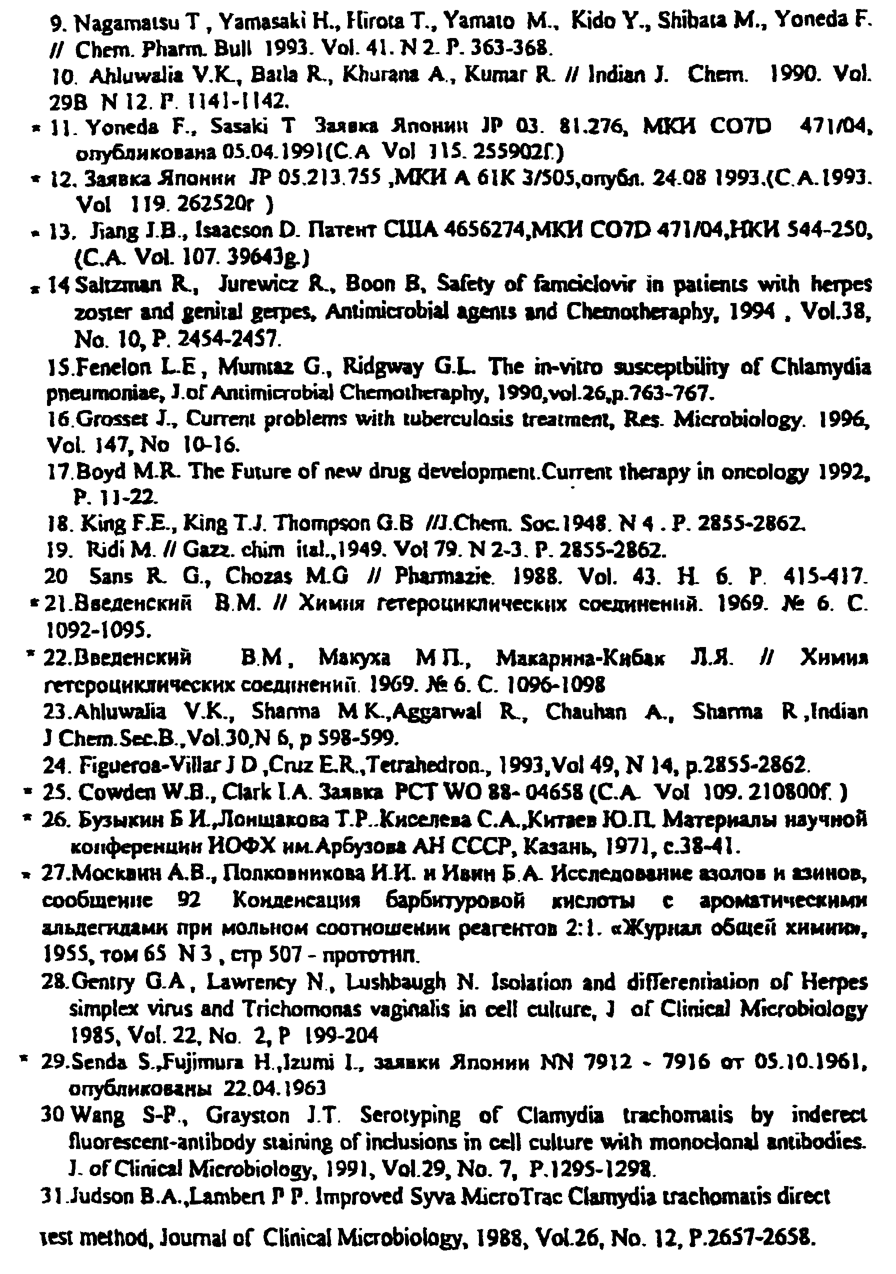

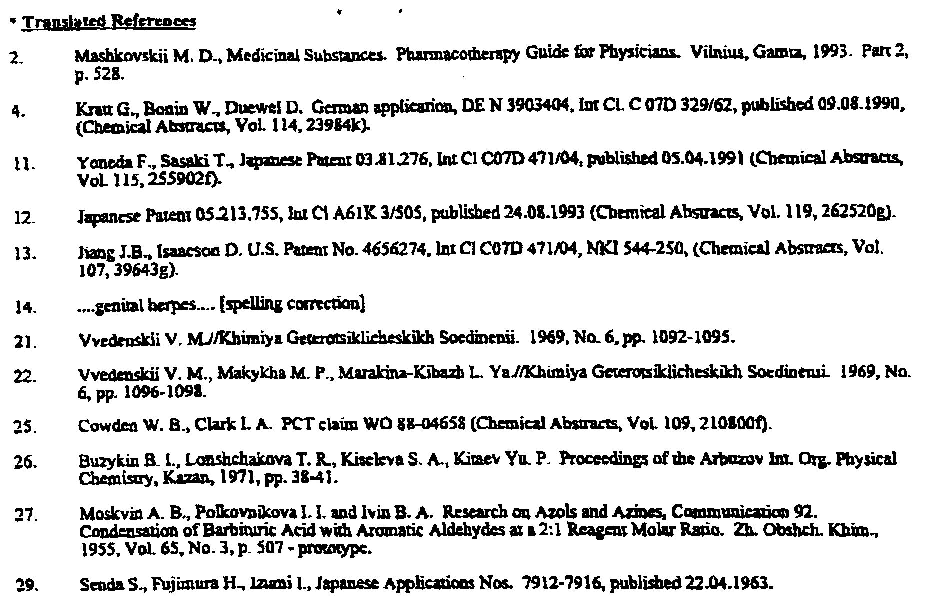

## Claims

1. Salts of 5,5'-arylidenebisbarbituric acids and of 5,5'-arylidenebis(2-thiobarbituric) acids and 5,5'-arylidenebis(2-thiobarbituric) acid of the general formula 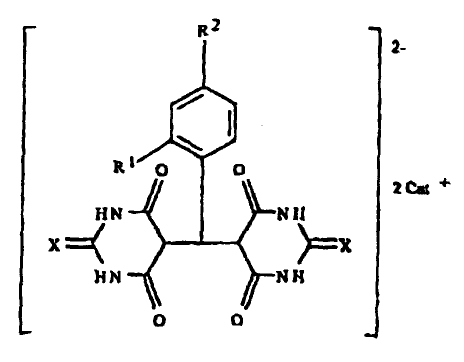 where
X is selected from the group OXYGEN, SULFUR,
R¹ is selected from the group HYDROGEN, the NITRO GROUP, or an ALKOXY GROUP,
R² is selected from the group HYDROGEN, the NITRO GROUP, the HYDROXYL GROUP, an ALKOXY GROUP, HALOGEN, [Tr. - "HYDROXYL" of Abstract is omitted]
Cat⁺ is selected from the group PROTON, or a PYRIDINE or 2-HYDROXYETHYLAMMONIUM CATION, which have antimicrobial, antiviral, immunomodulating, and anti-tumor activities.

2. The substance claimed in Claim 1, except that

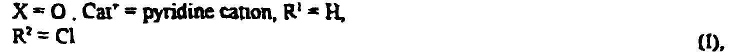

3. The substance claimed in Claim 1, except that

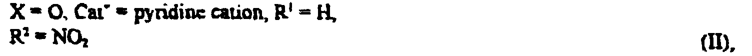

4. The substance claimed in Claim 1, except that

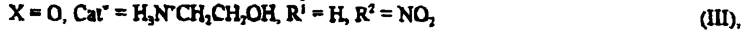

5. The substance claimed in Claim 1, except that

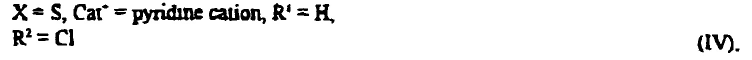

6. The substance claimed in Claim 1, except that

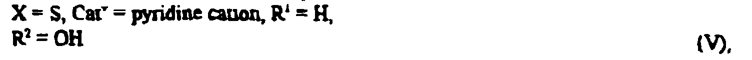

7. The substance claimed in Claim 1, except that

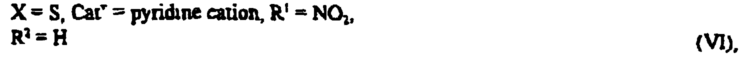

8. The substance claimed in Claim 1, except that

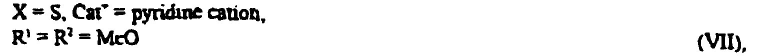

9. The substance claimed in Claim 1, except that

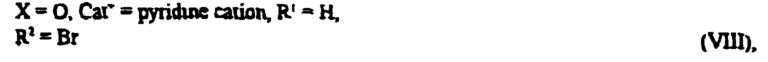

10. The substance claimed in Claim 1, except that

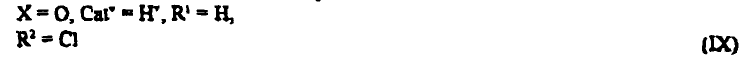

11. The substance claimed in Claim 1, except that
